# EUROPEAN PATENT APPLICATION

(11) **EP 3 311 728 A1**
(43) Date of publication of application: **25.04.2018**
(21) Application number: 16811278.7
(22) Date of filing: 28.03.2016
(51) Int. Cl.: A61B 1/00, G02B 23/24

(54) **INSERTION APPARATUS AND INSERTION DEVICE**

(30) Priority: 18.06.2015 JP 2015122964
(71) Applicant: Olympus Corporation, Hachioji-shi Tokyo 192-8507 (JP)
(72) Inventor: MASAKI, Yutaka, Tokyo 192-8507 (JP); MITAMURA, Yuki, Tokyo 192-8507 (JP)
(74) Representative: Schicker, Silvia
(86) International application number: PCT/JP2016/059915
(87) International publication number: WO 2016/203803

(57) **Abstract**

An insertion instrument includes an insertion section extending from a proximal side to a distal side along a longitudinal axis, and the insertion section includes an assistance tool attaching portion to which an assistance tool is attached. A dimension index indicating a dimension from a distal end of the insertion section is provided on an outer surface of the insertion section. A position index provided at a reference position, which is spaced from a proximal end of the assistance tool attached to the assistance tool attaching portion at a predetermined dimension to the proximal side, on the outer surface of the insertion section, the position index being an index indicating a position is the reference position.

## Description

### Technical Field

The present invention relates to an insertion instrument in which an assistance tool attaching portion for attaching an assistance tool is provided in an insertion section, and an insertion device including the insertion instrument and the assistance tool.

### Background Art

International Publication No. 2013/038720 discloses an endoscope device including an insertion section extending along a longitudinal axis, and an assistance tool (a spiral unit) which is attached to an assistance tool attaching portion provided in the insertion section. In the endoscope device, the assistance tool rotates about the longitudinal axis when a driving force is transmitted to the assistance tool with the assistance tool being attached to the assistance tool attaching portion. The rotation of the assistance tool while being pressed toward the inner peripheral side causes a propulsion force toward the distal side or the proximal side to act on the insertion section and the assistance tool.

### Summary of Invention

In the insertion device disclosed in, for example, International Publication No. 2013/038720, when the inside of a lumen is observed using the insertion device, an insertion section and an assistance tool attached to the assistance tool attaching portion are inserted into the lumen from an insertion opening (i.e., the mouth). The observation of the inside of the lumen may be performed while the overall length of the assistance tool in the direction along the longitudinal axis is located inside of the lumen. In this case, since the assistance tool does not project outside of the patient's body from the insertion opening of the lumen, it is difficult for a surgeon to ascertain the position of the assistance tool which is located inside of the lumen.

The present invention was designed to address the above problem, and an objective of the present invention is to provide an insertion instrument and an insertion device that allow a surgeon to easily ascertain the position of the assistance tool inside the lumen.

To solve the above mentioned problems, according to one aspect of the invention, an insertion instrument including: an insertion section including an assistance tool attaching portion to which an assistance tool is attached, the insertion section extending from a proximal side to a distal side along a longitudinal axis; a dimension index which is provided on an outer surface of the insertion section, and which indicates a dimension from a distal end of the insertion section; and a position index provided at a reference position, which is spaced from a proximal end of the assistance tool attached to the assistance tool attaching portion at a predetermined dimension to the proximal side, on the outer surface of the insertion section, the position index being an index indicating a position is the reference position.

### Brief Description of Drawings

FIG. 1 is a schematic diagram of an endoscope device according to a first embodiment,
FIG. 2 is a schematic diagram of a proximal portion of an insertion section according to the first embodiment,
FIG. 3 is a schematic diagram of a dimension index and a position index according to a first modification,
FIG. 4 is a schematic diagram of a dimension index and a position index according to a second modification,
FIG. 5 is a schematic diagram of a dimension index and a position index according to a third modification,
FIG. 6 is a schematic diagram of a dimension index and a position index according to a fourth modification,
FIG. 7 is a schematic diagram of a proximal portion of an insertion section according to a fifth modification,
FIG. 8 is a schematic view of a proximal portion of an insertion section according to a sixth modification, and
FIG. 9 is a schematic diagram of a proximal portion of an insertion section according to a seventh modification. Description of Embodiments

### (First Embodiment)

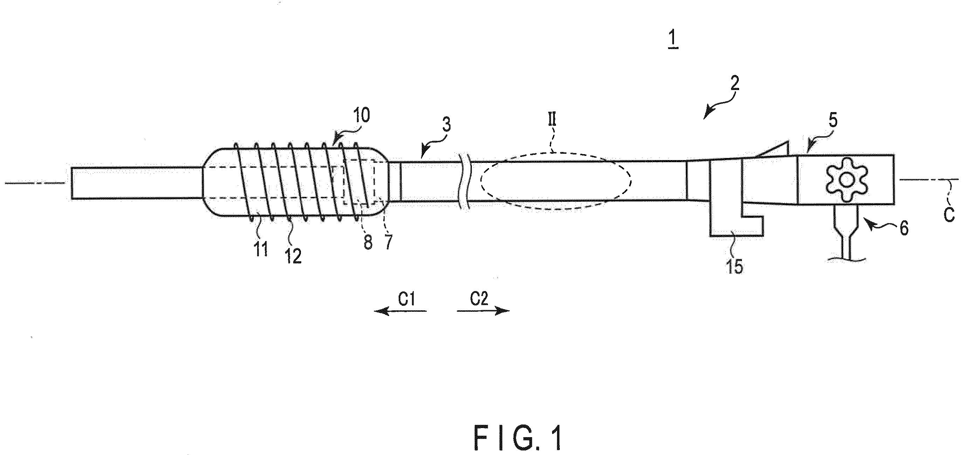

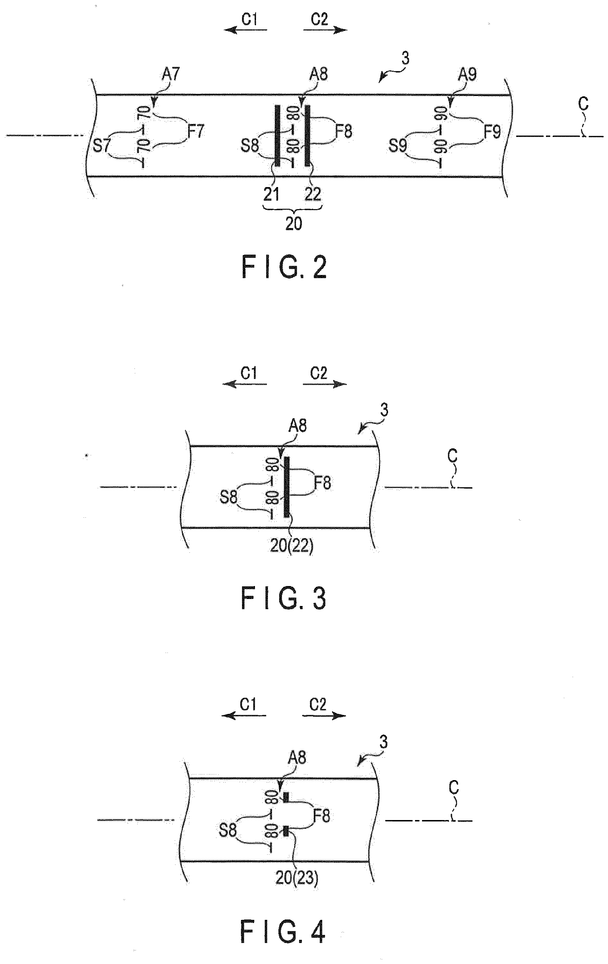

The first embodiment of the present invention will be explained with reference to FIG. 1 and FIG. 2. FIG. 1 shows an endoscope device 1 which is an insertion device according to the present embodiment.

As shown in FIG. 1, the endoscope device 1 includes an endoscope 2 which serves as an insertion instrument, and a spiral unit 10 which serves as an assistance tool. The endoscope 2 includes an insertion section 3, and the insertion section 3 has a longitudinal axis C. Herein, the direction along the longitudinal direction is defined as a longitudinal axis C. One side on the longitudinal axis C is defined as a distal side (indicated by arrow C1 in FIG. 1), and a side opposite to the distal side is defined as a proximal side (indicated by arrow C2 in FIG. 1). The insertion section 3 extends from the proximal side toward the distal side along the longitudinal axis C, and in the endoscope 2, an operation section 5 is provided on the proximal side with respect to the insertion portion 3. The endoscope 2 includes a universal cord 6 one end of which is connected to the operation section 5. The endoscope 2 is connected, via the universal cord 6, to a peripheral device unit that includes an image processing device (not shown in the drawings), a light source device (not shown in the drawings), a drive control device (not shown in the drawings), an operation input device (not shown in the drawings) such as a foot switch etc., and a display device (not shown in the drawings) such as a monitor, etc.

An imaging element (not shown in the drawings) such as a CCD is provided inside of the distal portion of the insertion section 3, and the imaging element captures an image of a subject through an observation window (not shown in the drawings) provided on the outer surface of the distal portion of the insertion portion 3. Furthermore, an image signal is transmitted to the image processing device via an imaging cable (not shown in the drawings) extending through the inside of the insertion section 3, the inside of the operation section 5, and the inside of the universal cord 6, and an imaging process is performed by the image processing device. Thus, an image of a subject is generated by the image processing device, and the generated image of the subject is displayed on the display device. Light emitted from the light source device is guided through a light guide extending through the inside of the insertion section 3, the inside of the operation section 5, and the inside of the universal cord 6. The guided light is emitted on the subject through an illumination window (not shown in the drawings) which is provided on the outer surface of the distal portion of the insertion portion 3.

The insertion section 3 includes a base portion 7, and a rotation body 8 that is rotatably attached to the base portion 7 in such a manner that the rotation body 8 rotates about the longitudinal axis C with respect to the base portion 7. The base portion 7 and the rotation body 8 constitute the assistance tool attaching portion to which a spiral unit (an assistance tool) 10 is detachably attached. When the spiral unit 10 is attached to the insertion section 3, the insertion section 3 is inserted through the spiral unit 10, and the outer peripheral side of the base portion 7 and the rotation body 8 are covered with the proximal portion of the spiral unit 10. The spiral unit 10 includes a cylindrical-shaped tube main body 11 extending along the longitudinal axis C, and a spiral fin 12 projecting on the outer peripheral surface of the tube main body 11 toward the outer peripheral side. The spiral fin 12 spirally extends around the longitudinal axis C.

A motor casing 15 is attached to the operation section 5, and an electric motor (not shown in the drawings), which is a drive source, is provided inside the motor casing 15. By performing an operation input with the operation input device (not shown in the drawings), electric power is supplied to the electric motor from the drive control device through electric lines (not shown in the drawings) extending through the inside of the operation section 5 and the inside of the universal cord 6, and the electric motor is driven. When the electric motor is driven, driving force is transmitted to a driving shaft (not shown in the drawings) extending between the motor casing 15 and the base portion 7 inside of the insertion portion 3, and the driving shaft rotates. Thus, the driving force is transmitted to the rotation body 8, and the rotation body 8 rotates about the longitudinal axis C with respect to the base portion 7. When the rotation body 8 rotates with the spiral unit 10 being attached to the assistance tool attaching portion (7, 8) of the insertion section 3, the spiral unit (an assistance tool) 10 rotates about the longitudinal axis C with the rotation body 8. In the present embodiment, a propulsion force in the distal side or the proximal side acts on the insertion section 3 and the spiral unit 10 when the spiral unit 10 rotates with pressing pressure acting on the spiral fin 12 toward the inner peripheral side.

FIG. 2 shows the configuration of the proximal portion of the insertion section 3 (the range II in FIG. 1). As shown in FIG. 2, a plurality of dimension indices Ai (i = 1, 2, ...) are provided on the outer surface of the insertion section 3. Each of the dimension indices Ai is an index indicating a dimension from the distal end of the insertion section 3. For example, the dimension index A7 is an index indicating a position that is 70 cm (700 mm) from the distal end of the insertion section 3 toward the proximal side. The dimension indices Ai are spaced from each other in the direction along the longitudinal axis C (the longitudinal direction); in the present embodiment, the dimension indices Ai are spaced every 10 cm (100 mm). Accordingly, each of the dimension indices Ai is placed at a position 10 × i cm (i = 1, 2, ...) from the distal end of the insertion section 3 to the proximal side. Each of the dimension indices Ai consists of a value Fi (i = 1, 2, ...), and a scale line Si (i = 1, 2, ...) that is a first line. In one example, when the spiral unit 10 is attached to the assistance tool attaching portion (7, 8), the distal end of the spiral unit 10 is located at a position that is 20 cm from the distal end of the insertion section 3 toward the proximal side, and the proximal end of the spiral unit 10 is located at a position that is 40 cm from the distal end of the insertion section 3 to the proximal side. In this case, the spiral unit 10 extends within the range where the dimension from the distal end of the insertion section 3 to the proximal side falls under the range between 20 cm and 40 cm.

A position index 20 is provided on the outer surface of the proximal portion of the insertion section 3. The position index 20 is located at a reference position that is spaced from the proximal end of the spiral unit (an assistance tool) 10 at a predetermined dimension D0 to the proximal side, with the spiral unit 10 being attached to the assistance tool attaching portion (7, 8), and the position index 20 becomes an index indicating that the position is a reference position. In one example, a predetermined dimension DO is 40 cm (400 mm), and a position that is 40 cm (400 mm) from the proximal end of the spiral unit 10 which is attached to the assistance tool attaching portion (7, 8) toward the proximal side is defined as a reference position. Accordingly, the position index 20 located at a reference position is separated from the range where the spiral unit (the assistance tool) 10 attached to the assistance tool attaching portion (7, 8) of the insertion section 3 rotates (actuates), in the direction along the longitudinal axis C. In the present embodiment, the position index 20 consists of emphasis lines 21 and 22 that are second lines. The position index 20 may be formed in the same color as or in a different color from the dimension indices Ai. In the present embodiment, the emphasis lines (the second lines) 21 and 22 of the position index 20 are formed thicker than the scale lines (the first lines) Si of the dimension indices Ai. Accordingly, the dimension of each of the emphasis lines (the second lines) 21 and 22 in the direction along the longitudinal axis C is larger than the dimension of each of the scale lines (the first lines) Si in the direction along the longitudinal axis C

Furthermore, in the present embodiment, the reference position (a position that is separated from the proximal end of the spiral unit 10 at a predetermined dimension DO to the proximal side) matches the position that is 80 cm (800 mm) from the distal end of the insertion section 3 on to proximal side. Thus, the dimension index A8, which is one of the plurality of dimension indices Ai, is located at the reference position. In the present embodiment, the position index 20 is formed by placing the emphasis line 21 adjacently to the distal side of the dimension index A8 and the emphasis line 22 adjacently to the proximal side of the dimension index A8, and the dimension index A8 is located between the emphasis lines 21 and 22 in the longitudinal direction.

Next, the function and advantageous effects of the endoscope (an insertion instrument) 2 and the endoscope device (an insertion device) 1 according to the present embodiment are explained. When the inside of a lumen, such as an intestine lumen, etc. is observed using the endoscope device 1, the spiral unit (an assistance tool) 10 is attached to the assistance tool attaching portion (7, 8) of the insertion section 3, and the insertion section 3 and the spiral unit 10 are inserted into the lumen. At this time, the insertion section 3 and the spiral unit 10 are inserted from an insertion opening, such as the mouth, into the lumen. Then, an operation input is performed by the operation input device (not shown in the drawings) with the spiral unit 10 being located inside of the lumen, the electric motor (not shown in the drawings) is driven, and the driving force is transmitted to the spiral unit 10 as described above. Thus, the spiral unit 10 rotates about the longitudinal axis C. When the spiral unit 10 rotates with the spiral fin 12 being pressed toward the inner peripheral side by the lumen wall, the propulsion force toward the distal side or the proximal side (one side of the direction along the longitudinal axis C) acts on the insertion section 3 and the spiral unit 10. The movability of the insertion section 3 in the lumen is improved by the propulsion force.

In the present embodiment, the position index 20 is provided at the reference position that is separated from the proximal end of the spiral unit (an assistance tool) 10 at a predetermined dimension DO to the proximal end, and the reference position is indicated by the position index 20. Thus, during the observation using the endoscope device 1, the surgeon can ascertain the position of the spiral unit 10 based on the position index 20. During the observation using the endoscope device 1, the overall length of the spiral unit 10 in the direction along the longitudinal axis C may be located inside of the lumen; even in this case, the surgeon can appropriately ascertain the position of the spiral unit 10 inside of the body lumen based on the position index 20 located outside the body.

For example, the insertion section 3 and the spiral unit 10 (the assistance tool) are inserted into the lumen from the mouth (an insertion opening) using the endoscope device 1 in which a position index 20 is provided at a reference position that is 40 cm (a predetermined dimension D0) from the proximal end of the spiral unit 10 to the proximal side. Herein, the dimension in the lumen (the esophagus) from the mouth (the insertion opening) to the stomach is usually about 40 cm (400 mm), with slight differences among subjects. Accordingly, while the insertion section 3 and the spiral unit (the assistance tool) 10 are moving to the distal side in the lumen, the surgeon appropriately ascertains that the proximal end of the spiral unit 10 reaches the stomach through the esophagus inside of the lumen, based on the move of the position index 20 to the region of near the mouth (the insertion opening) toward the distal side.

Since the position of the spiral unit (the assistance tool) 10 in the inside of the lumen is ascertained based on the position index 20 as described above, there is no necessity of checking the position of the spiral unit 10 by, for example, X-ray radioscopy, etc. Thus, the position of the spiral unit 10 in the lumen can be easily and appropriately ascertained in the present embodiment. For this reason, the workability is improved in the observation of a body cavity using the endoscope device 1.

### (Modifications)

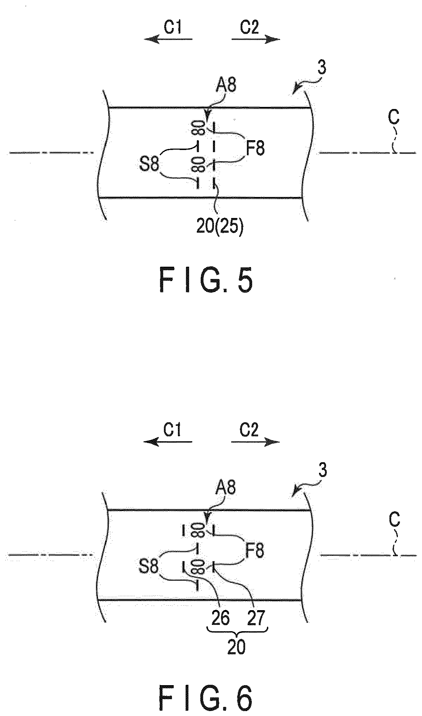

The indicating state of the position index 20 is not limited to that in the first embodiment. For example, in the first modification shown in FIG. 3, a position index 20 is formed only by an emphasis line 22 adjacently provided on the proximal side of the dimension index A8. In the second modification shown in FIG. 4, an emphasis pattern 23 is adjacently provided on the proximal side of the dimension index A8, and a position index 20 is formed only by the emphasis pattern 23. In the third modification shown in FIG. 5, an emphasis dashed line 25 is adjacently provided on the proximal side of the dimension index A8, and a position index 20 is formed only by the emphasis dashed line 25. In the fourth modification shown in FIG. 6, an emphasis pattern 26 is adjacently provided on the distal side of the dimension index A8, an emphasis pattern 27 is adjacently provided on the proximal side of the dimension index A8, and the dimension index A8 is located between the emphasis pattern 26 and the emphasis pattern 27 in the direction along the longitudinal axis C. And, a position index 20 is formed by the emphasis pattern 26 and the emphasis pattern 27. In each of the first to fourth modifications, the dimension index A8, which is one of the dimension indices Ai, and the position index 20 are located at the reference position that is spaced from the proximal end of the spiral unit 10 attached to the assistance tool attaching portion (7, 8) at a predetermined dimension D0 to the proximal side.

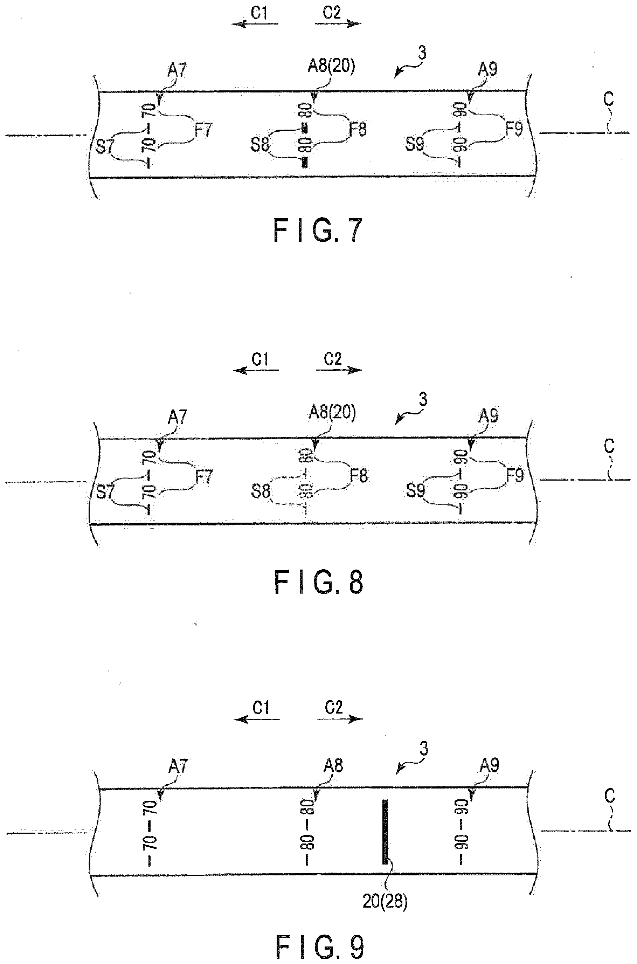

In a configuration in which one of the plurality of dimension indices Ai (for example, A8) is located at the reference position, a position index 20 may be formed by indicating the dimension index (for example, A8) that is located at the reference position in an indicating state different from that of the other dimension indices (for example, Ai other than A8). For example, in the fifth modification shown in FIG. 7, the scale line S8 of the dimension index A8 located at the reference position that is spaced from the proximal end of the spiral unit 10 at a predetermined dimension DO to the proximal side is formed thicker than the scale lines (Si other than S8) of the other dimension indices (Ai other than A8). The dimension index A8 may serve also as the position index 20 indicating the reference position, by forming only the scale line S8 of the dimension index A8 to be thick. In the sixth modification shown in FIG. 8, the dimension index A8 located at the reference position is indicated in a dashed line, whereas the other dimension indices (Ai other than A8) are indicated in solid lines. By indicating only the dimension index A8 in a dashed line, the dimension index A8 may serve also as the position index 20 indicating the reference position. In one modification, a position index (20) may be formed by a dimension index (for example, A8) located at a reference position by indicating the dimension index (for example, A8) located at the reference position in a color different from the color of the other dimension indices (for example, Ai other than A8).

In the foregoing embodiments and modifications, the dimension index A8 is located at the reference position that is spaced from the proximal end of the spiral unit 10 at a predetermined dimension DO to the proximal side; however, the dimension indices Ai other than the dimension index A8 may be located at the reference position. For example, in a configuration in which the dimension index A7 is located at the reference position, the position that is 70 cm (700 mm) from the distal end of the insertion section 3 to the proximal side corresponds to the reference position that is spaced from the proximal end of the spiral unit 10 at a predetermined dimension DO to the proximal side.

A configuration in which none of the dimension indices Ai is located at the reference position at which the position index 20 is located may be possible. For example, in the seventh modification shown in FIG. 9, an emphasis line 28 is provided in an extended portion between the dimension index A8 and the dimension index A9 in a direction along the longitudinal axis C, and a position index 20 is formed by the emphasis line 28. Then, the position index 20 is located at the reference position that is spaced from the proximal end of the spiral unit 10 at a predetermined dimension DO to the proximal side. Accordingly the position index 20 is separated from each of the dimension indices Ai in the longitudinal direction, and none of the dimension indices Ai are located at the reference position. In this case, for example, the position that is 85 cm (850 mm) from the distal end of the insertion section 3 to the proximal side corresponds to the reference position that is spaced from the proximal end of the spiral unit 10 at a predetermined dimension D0 to the proximal side.

In the foregoing embodiment and modifications, the spiral unit (10) was explained as an example of an assistance tool that is attached to the insertion section (3); however, an assistance tool is not limited to the spiral unit (10). For example, an assistance tool to which a actuation section which is movably provided with respect to the insertion section (3) in a direction along the longitudinal axis C may be attached to the insertion section (3). In the foregoing embodiment and modifications, the endoscope (2) was explained as an example of an insertion instrument; however, an insertion instrument is not limited to an endoscope (2). For example, the foregoing configuration may be applied to an insertion device in which a manipulator is adopted as an insertion instrument.

In the foregoing embodiment and modifications, the insertion instrument (2) includes the insertion section (3) extending from the proximal side to the distal side along the longitudinal axis, and the assistance tool attaching portion (7, 8) to which the assistance tool (10) is attached is provided in the insertion section (3). The dimension indices (Ai) are provided on the outer surface of the insertion section (3) as indices indicating dimensions from the distal end of the insertion section (3). On the outer surface of the insertion section (3), a position index (20) is provided at a reference position that is spaced from the proximal end of an assistance tool (10) attached to an assistance tool attaching portion (7, 8) at a predetermined dimension D0 to the proximal side, and the position index (20) is an index that indicates the reference position.

Although the embodiments, etc. of the present invention have been described above, the present invention is not limited to the above-described embodiments, etc., and, needless to say, various modifications can be made without departing from the spirit of the invention.

## Claims

1. An insertion instrument comprising:
an insertion section including an assistance tool attaching portion to which an assistance tool is attached, the insertion section extending from a proximal side to a distal side along a longitudinal axis;
a dimension index which is provided on an outer surface of the insertion section, and which indicates a dimension from a distal end of the insertion section; and
a position index provided at a reference position, which is spaced from a proximal end of the assistance tool attached to the assistance tool attaching portion at a predetermined dimension to the proximal side, on the outer surface of the insertion section, the position index being an index indicating a position is the reference position.

2. The insertion instrument according to claim 1, wherein
the dimension index is a plurality of dimension indices which are spaced from each other in a direction along the longitudinal axis, and
one of the plurality of dimension indices is located at the reference position.

3. The insertion instrument according to claim 2, the dimension index located at the reference position forms the position index by being indicated in an indicating state different from that of the other dimension indices.

4. The insertion instrument according to claim 1, wherein the position index is formed in a same color as the dimension index.

5. The insertion instrument according to claim 1, wherein
the dimension index has a first line, and
the position index includes a second line which is formed thicker than the first line of the dimension index.

6. The insertion instrument according to claim 1, wherein the reference position is a position that is 400 mm from the proximal end of the assistance tool attached to the assistance tool attaching portion to the proximal side.

7. An insertion device comprising:
the insertion instrument according to claim 1; and
the assistance tool which is attached to the assistance tool attaching portion of the insertion section.

8. The insertion device according to claim 7, the position index is located so as to be separated from a range, in which the assistance tool attached to the insertion section is actuated, in a direction along the longitudinal axis.
